(19) Europäisches Patentamt
European Patent Office
Office européen des brevets

(11) **EP 3 380 198 B1**

(12) **FASCICULE DE BREVET EUROPEEN**

(45) Date de publication et mention
de la délivrance du brevet:
**24.06.2020 Bulletin 2020/26**

(21) Numéro de dépôt: **16813005.2**

(22) Date de dépôt: **22.11.2016**

(51) Int Cl.:
*A61Q 1/02* (2006.01)     *A61K 8/02* (2006.01)
*A61K 8/25* (2006.01)     *A61K 8/26* (2006.01)
*A61K 8/19* (2006.01)     *A61Q 19/00* (2006.01)

(86) Numéro de dépôt international:
**PCT/FR2016/053054**

(87) Numéro de publication internationale:
**WO 2017/089701 (01.06.2017 Gazette 2017/22)**

(54) **COMPOSITION COSMÉTIQUE COMPRENANT AU MOINS UNE POUDRE PRÉSENTANT UNE FAIBLE CONDUCTIVITÉ THERMIQUE**

KOSMETISCHE ZUBEREITUNG ENTHALTEND EIN PULVER, DAS EINE NIEDRIGE WÄRMELEITFÄHIGKEIT AUFWEIST

COSMETIC COMPOSITION COMPRISING AT LEAST A POWDER EXHIBITING A LOW THERMAL CONDUCTIVITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.11.2015 FR 1561247**

(43) Date de publication de la demande:
**03.10.2018 Bulletin 2018/40**

(73) Titulaire: **Chanel Parfums Beauté**
**92200 Neuilly-sur-Seine (FR)**

(72) Inventeurs:
• **DE CLERMONT-GALLERANDE, Helene**
**94300 Vincennes (FR)**

• **GUARILLOFF, Philippe**
**Princeton**
**NJ 08540 (US)**

(74) Mandataire: **Plasseraud IP**
**66, rue de la Chaussée d'Antin**
**75440 Paris Cedex 09 (FR)**

(56) Documents cités:
| | |
|---|---|
| EP-A1- 1 829 522 | WO-A2-2012/027194 |
| JP-A- 2001 172 125 | US-A1- 2010 003 340 |
| US-A1- 2013 039 869 | US-A1- 2014 050 672 |
| US-A1- 2014 242 127 | |

**Description**

La présente invention a pour objet l'utilisation d'au moins une poudre présentant une conductivité thermique inférieure ou égale à 2 W.m$^{-1}$.K$^{-1}$ pour améliorer le confort thermique conféré par une composition cosmétique de maquillage ou de soin de la peau.

La peau constitue une barrière qui protège l'organisme contre les agressions extérieures telles que les variations de température, l'humidité, les rayons ultraviolets (UV), etc. L'exposition répétée à ces facteurs néfastes entraine un vieillissement prématuré de la peau. Il est par exemple connu que la chaleur accélère le vieillissement cutané en augmentant la température de la peau, provoquant diverses réponses inflammatoires et dégradant le collagène dans le derme en induisant une augmentation de collagénases.

Afin d'éviter ou de prévenir les dommages cutanés causés les rayonnements ultraviolets (UV), il est connu d'utiliser des filtres solaires dans les compositions cosmétiques. Toutefois, bien que ces filtres protègent efficacement la peau des rayonnements UV, ils n'empêchent pas la sensation de chaleur induite par ces rayonnements.

De la même manière, les peaux exposées à des climats froids ont tendance à rougir, à tirailler et à générer des inflammations. L'application de crèmes particulièrement hydratantes permet certes de soulager ces symptômes a posteriori, mais ne permet pas de protéger la peau contre ces agressions.

Il serait par conséquent souhaitable de proposer des compositions cosmétiques permettant d'améliorer le confort thermique de la peau, c'est-à-dire permettant de former sur la peau une couche barrière isolante protectrice, notamment en cas d'exposition à des climats chauds et humides ou froids et secs.

La demande WO2014/133957 de la société ELC Management LLC tente de répondre à cette problématique au moyen de compositions cosmétiques mettant en œuvre des matériaux capables de changer des phases (solide/liquide) selon les conditions de températures extérieures. Quand la température augmente, le matériau absorbe la chaleur et passe à l'état liquide, puis lorsque la température baisse à nouveau le matériau libère la chaleur stockée et repasse à l'état solide, protégeant ainsi la peau des variations de température. Toutefois, l'utilisation de ce type de composés changeant de phase pose des problématiques importantes de formulation puisqu'ils sont sensibles à la température.

On reste donc à la recherche d'une solution technique plus simple à mettre en œuvre permettant de contrôler les variations de températures de la peau exposée à des conditions climatiques rigoureuses, de manière à en améliorer le confort thermique.

Le but de la présente invention est donc de proposer une composition de maquillage ou de soin de la peau capable de protéger la peau des variations thermiques extérieures et facile à mettre en œuvre.

Les inventeurs ont découvert que la mise en œuvre de poudres présentant une conductivité thermique particulière permettait d'améliorer le confort thermique conféré par une composition cosmétique de maquillage ou de soin de la peau.

L'invention a ainsi pour objet l'utilisation d'au moins une poudre présentant une conductivité thermique inférieure ou égale à 2 W.m$^{-1}$.K$^{-1}$, de préférence comprise entre 0 et 2 W.m$^{-1}$.K$^{-1}$, plus préférentiellement entre 0,1 et 1,52 W.m$^{-1}$.K$^{-1}$ pour améliorer le confort thermique conféré par une composition cosmétique de maquillage ou de soin de la peau.

Il est en effet du mérite de la demanderesse d'avoir mis en évidence que certaines poudres, par ailleurs connues dans le domaine cosmétique, pouvaient permettre de constituer une barrière protectrice sur la peau du fait de leur faible conductivité thermique.

Description des figures

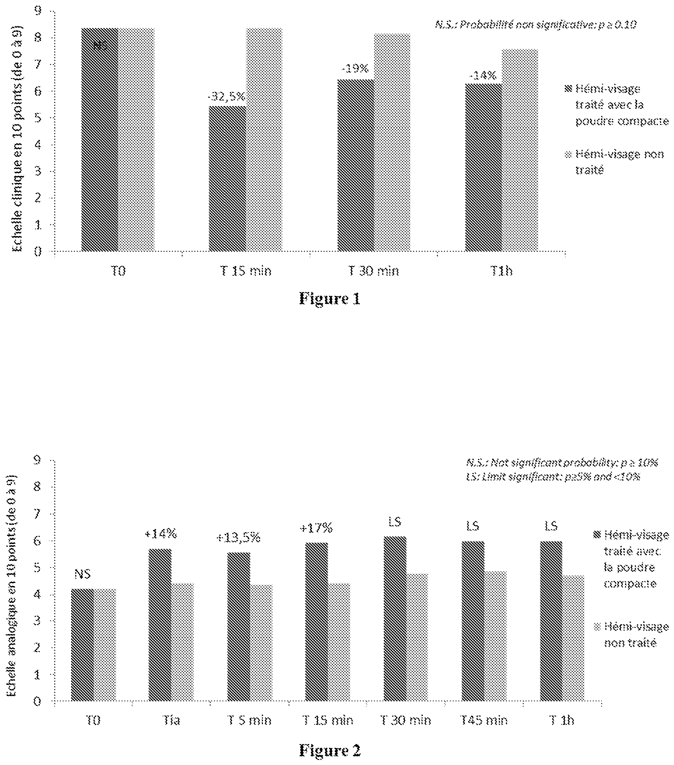

Les figures 1 et 2 illustrent respectivement l'évaluation par un technicien de la visibilité des pores de la peau et l'autoévaluation du confort ressenti par l'utilisatrice dans des conditions chaudes et humides (exprimées dans les deux cas en pourcentage de variation de la moitié du visage traitée par rapport à la moitié non traitée).

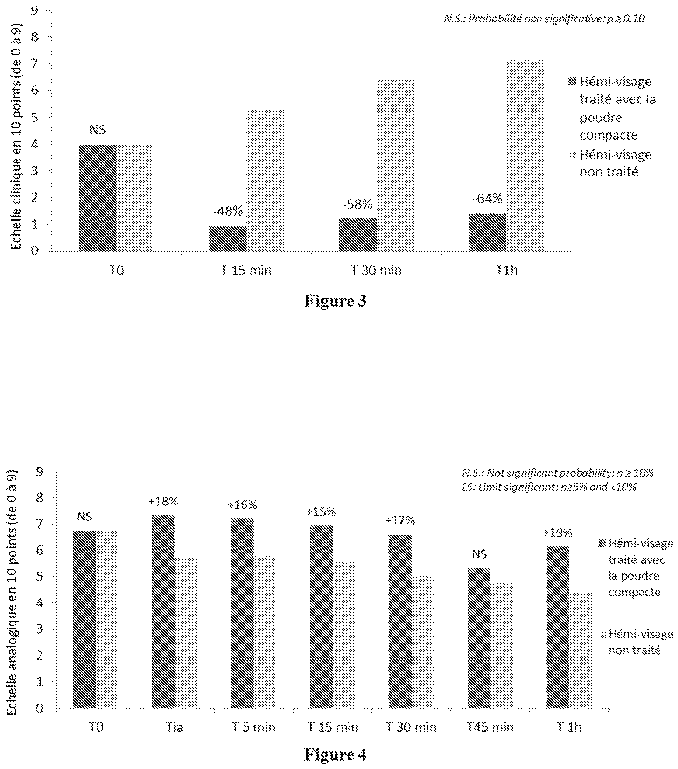

Les figures 3 et 4 illustrent l'évaluation clinique par un technicien de la visibilité des rougeurs de la peau et l'autoévaluation du confort ressenti par l'utilisatrice dans des conditions froides et sèches (exprimées dans les deux cas en pourcentage de variation de la moitié du visage traitée par rapport à la moitié non traitée).

Confort thermique

On estime que la zone de confort pour l'humain se situe dans des conditions standard de température de l'ordre de 20°C, et d'humidité relative d'environ 50%.

Au sens de la présente demande, on entend que le confort thermique conféré par une composition cosmétique est amélioré lorsque l'application de la composition sur la peau permet de l'isoler, au moins en partie, des fluctuations de température et d'humidité de l'environnement extérieur, par exemple permet de diminuer la visibilité des pores observée dans des conditions représentatives d'une chaleur humide (par exemple 30°C, et 75% d'humidité relative), de diminuer les rougeurs observées dans les conditions d'un froid sec (par exemple 7°C, 40% d'humidité relative), et/ou d'améliorer la sensation de confort de la peau ressentie par l'utilisatrice.

Poudre présentant une faible conductivité thermique

La poudre mise en œuvre dans l'invention présente une faible conductivité thermique, en particulier inférieure ou égale à 2 W.m$^{-1}$.K$^{-1}$, de préférence comprise entre 0 et 2 W.m$^{-1}$.K$^{-1}$, plus préférentiellement entre 0,1 et 1,5 W.m$^{-1}$.K$^{-1}$.

La conductivité thermique peut être mesurée par toute méthode connue de l'homme du métier.

Dans le cadre de la présente demande, elle peut par exemple être calculée par le biais de la diffusivité thermique à laquelle elle est liée par la relation suivante :

$$\lambda(T) = \rho(T).C_p(T).a(T)$$

avec

$\lambda(T)$ = conductivité thermique en W.m$^{-1}$ .K$^{-1}$

$p(T)$ = masse volumique en g.m$^{-3}$

$C_p(T)$ = chaleur spécifique en J.g$^{-1}$.K$^{-1}$

$a(T)$ = diffusivité thermique en cm$^2$.s$^{-1}$

$T$ = température

La diffusivité thermique $a(T)$ peut être mesurée au moyen de l'appareil laser NETZSCH LFA 467 HyperFlash®.

Un échantillon est placé dans un porte-échantillon spécial pour poudres qui se trouve à l'intérieur d'un four maintenu à température constante (température ambiante, 20°C). Une de ses faces est illuminée par des pulses (de l'ordre de la milliseconde) émis par une lampe flash au xénon, ce qui assure un chauffage uniforme de la face avant.

La température de la face arrière est mesurée, en fonction du temps, à l'aide d'un capteur de mesure infrarouge (InSb ou MCT). L'acquisition et l'évaluation des données sont réalisées à l'aide du logiciel 32/64 bits Proteus® - Windows®. Le taux d'acquisition est de 2 MHz. Le LFA 467 HyperFlash® fonctionne en conformité avec les normes ASTM E-1461, DIN 30905 et DIN EN 821.

La diffusivité thermique de chaque échantillon est mesurée cinq fois, et on retient la valeur moyenne.

En l'absence de pertes thermiques de l'échantillon, la température devrait augmenter de manière linéaire. Dans une situation réelle, l'enregistreur mesure un pic de température suivi d'un retour à la température du four. Le temps t nécessaire pour que la face arrière atteigne la moitié de la température de pic (par rapport à la température du four), permet de déterminer la diffusivité thermique suivant :

$$a = \frac{1,37.d^2}{t.\pi^2},$$

d étant l'épaisseur de l'échantillon.

Il est alors possible de calculer la conductivité thermique grâce à la masse spécifique et la chaleur spécifique.

La chaleur spécifique ($C_p$, chaleur spécifique à pression constante) peut être déterminée par calorimétrie différentielle (DSC) par exemple sur un appareil 204 *F1 Phenix*®. La mesure est effectuée sous une atmosphère d'azote dynamique (20 ml/min). L'échantillon testé a une masse d'environ 6 mg. Des creusets en aluminium à basse pression ont été utilisés pour ces tests. Chaque échantillon est chauffé entre 20°C et 40°C à une vitesse de chauffe de 10°C/min.

La masse volumique à la température ambiante est calculée par la masse et le volume de la poudre.

La poudre présentant une conductivité thermique inférieure ou égale à 2 W.m$^{-1}$.K$^{-1}$ peut être toute particule de forme quelconque (notamment sphérique ou lamellaire), minérale ou organique, insoluble dans une composition cosmétique.

La poudre présentant une conductivité thermique inférieure ou égale à 2 W.m$^{-1}$.K$^{-1}$ peut par exemple être la cellulose, le mica, le talc, la silice, l'alumine, les silicates d'aluminium, notamment les borosilicates d'aluminium comme les billes de verre (par exemple Luxsil® de Presperse), le silicate de magnésium et d'aluminium comme le fluorphlogopite, la bentonite, le phosphate de calcium comme l'hydroxyapatite, le sulfate de magnésium, le carbonate de magnésium, le kaolin, le nitrure de bore, les poudres de PMMA, les polyamides, les résines de silicone telles que les polymethylsilsesquioxanes, les poudres d'élastomère de silicone éventuellement enrobées par une résine de silicone, et leurs mélanges.

De préférence, la charge présentant une conductivité thermique inférieure ou égale à 2 W.m$^{-1}$.K$^{-1}$ peut être choisie parmi le nitrure de bore, le talc, le mica, les microsphères de céramique enrobées de TiO$_2$ et de SiO$_2$, les billes de verre, la porcelaine, et leurs mélanges.

De manière encore plus préférée, la charge présentant une conductivité thermique inférieure ou égale à 2 W.m$^{-1}$.K$^{-1}$ est choisie parmi le nitrure de bore et les microsphères de céramique enrobées de TiO$_2$ et de SiO$_2$, et leurs mélanges.

Parmi les microsphères de céramique enrobées de TiO$_2$ et de SiO$_2$ utilisables dans le cadre de l'invention, on peut notamment citer les Ronaflair LDP White commercialisés par la société Merck, et parmi les nitrures de bore utilisables dans le cadre de l'invention, on peut notamment citer les Softouch CC6059 BN commercialisés par la société Momentive.

La poudre de faible conductivité thermique peut également être enrobée d'oxydes métalliques pour lui conférer une couleur ou un effet visuel particulier, sous réserve de ne pas augmenter significativement sa conductivité thermique.

Dans le cadre de la présente invention, la poudre de faible conductivité thermique devrait être utilisée en une teneur suffisante pour procurer à la peau le confort thermique souhaité.

En particulier, dans une composition cosmétique sous forme de poudre, la charge de faible conductivité thermique peut représenter 10 à 90% en poids, de préférence 20 à 80% en poids, et plus préférentiellement 35 à 70% en poids, par rapport au poids total de la composition cosmétique la comprenant.

Dans une composition cosmétique fluide, la charge de faible conductivité thermique peut représenter 0,1 à 50% en poids, de préférence 1 à 20% en poids, et plus préférentiellement 2 à 15% en poids, par rapport au poids total de la composition cosmétique la comprenant.

Composition cosmétique

La poudre présentant une conductivité thermique inférieure ou égale à 2 W.m$^{-1}$.K$^{-1}$ est notamment mise en œuvre dans une composition cosmétique de maquillage ou de soin de la peau.

La composition peut notamment se présenter sous la forme d'une poudre, d'une émulsion, d'une microémulsion, d'une nanoémulsion, d'une suspension, d'une solution, d'une lotion, d'une crème, d'un gel aqueux ou hydroalcoolique, d'une mousse, d'un sérum, ou d'une dispersion pour aérosol, d'une composition anhydre.

Dans le cas d'une émulsion, il peut s'agir d'une émulsion eau-dans-huile ou huile-dans-eau.

Dans le cas d'une poudre, il peut s'agir d'une poudre libre, compacte ou coulée, ou encore d'une composition obtenue par un procédé de type « slurry » c'est-à-dire par dispersion d'une phase pulvérulente dans une phase solvant volatile, puis évaporation de la phase solvant volatile.

En particulier, le produit de l'invention peut se présenter sous forme de crème de jour, de base de teint, de fond de teint, de produit anti-cernes, ou contours des yeux, d'ombre à paupières, de produit de maquillage ou de soin du corps.

Selon un mode préféré de réalisation, la composition est une composition de maquillage se présentant sous la forme d'une poudre libre, compacte ou coulée, de préférence d'une poudre compacte.

Selon un autre mode préféré de réalisation, la composition se présente sous forme d'une composition fluide, par exemple d'un fond de teint fluide anhydre ou d'une émulsion huile-dans-eau ou eau-dans-huile.

Charge additionnelle

La composition selon l'invention peut comprendre en outre au moins une charge additionnelle autre que la poudre présentant une conductivité thermique inférieure ou égale à 2 W.m$^{-1}$.K$^{-1}$ précédemment décrites.

Par « charge », on entend toute particule de forme quelconque (notamment sphérique ou lamellaire), minérale ou organique, insoluble dans la composition.

La charge additionnelle peut être présente dans la composition en une teneur allant de 1 à 50% en poids, de préférence de 5 à 40% en poids, et plus préférentiellement de 10 à 30% en poids, par rapport au poids total de la composition.

De préférence, la composition selon l'invention ne contient que des charges de conductivité thermique inférieure à 2 W.m$^{-1}$.K$^{-1}$.

Huiles

La composition peut également comprendre au moins une huile.

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Comme huiles pouvant être utilisées dans les compositions selon l'invention, on peut notamment citer : les hydrocarbures linéaires ou ramifiés d'origine minérale ou synthétique, les (poly)esters et (poly)éthers de synthèse et en particulier les (poly) esters d'acides en $C_6$-$C_{20}$ et d'alcools en $C_6$-$C_{20}$ avantageusement ramifiés, tels que l'isononanoate d'isononyle, les huiles végétales, les acides gras ramifiés et/ou insaturés, les alcools gras ramifiés et/ou insaturés, les huiles de silicone, les huiles fluorosiliconées, les huiles fluorées, ainsi que leurs mélanges.

Parmi ces huiles, on préfère que la composition selon l'invention comprenne au moins une huile de silicone.

On entend par "huile de silicone", une huile comprenant au moins un atome de silicium, et notamment au moins un groupe Si-O. L'huile de silicone peut être volatile ou non volatile.

Comme huile de silicone non volatile, on peut notamment citer les polydiméthylsiloxanes renfermant au moins 8 atomes de silicium, les polyalkylméthylsiloxane dont la chaîne alkyle renferme de 8 à 20 atomes de carbone et les huiles identifiées par le nom INCI phenyl trimethicone.

Comme huile de silicone volatile, on peut citer notamment certaines diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, les composés identifiés par les noms INCI methyl trimethicone et caprylyl methicone et leurs mélanges.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camellia.

On entend par "huile hydrocarbonée", une huile contenant uniquement des atomes d'hydrogène et de carbone. Des exemples d'huiles hydrocarbonées non volatiles sont le polybutène, le polyisobutène hydrogéné et le polydécène hydrogéné. Les huiles hydrocarbonées volatiles peuvent être choisies parmi les huiles hydrocarbonées contenant de 8 à 16 atomes de carbone, et notamment les alcanes ramifiés en $C_8$-$C_{16}$ (appelées aussi isoparaffines) comme l'isododécane, l'isodécane ou l'isohexadécane, et les alcanes linéaires volatils en $C_{11}$-$C_{14}$ comme par exemple le Cetiol Ultimate commercialisé par la société BASF, ou le Vegelight 1214 LC commercialisé par la société Biosynthis.

On entend par « huiles fluorées », une huile contenant au moins un atome de fluor, telle que le nonafluorométhoxybutane ou le perfluorométhyl-cyclopentane, le perfluorodiméthylcyclohexane, le perfluoroperhydrophénanthrène, la perfluorodécaline, et leurs mélanges, sans que cette liste ne soit limitative.

Cire et gélifiant lipophile

La composition selon l'invention peut en outre comprendre au moins une cire et/ou au moins un gélifiant lipophile.

Par « cire », on entend un corps gras ayant une température de fusion supérieure à 30°C et généralement inférieure à 100°C, qui est liquide dans les conditions de préparation de la composition et présente à l'état solide une organisation cristalline anisotrope. Des exemples de cires sont notamment les cires végétales, minérales ou synthétiques, ces dernières pouvant avantageusement être des cires hydrocarbonées ou siliconées. On peut ainsi mentionner les cires de Carnauba, de Candelilla, de riz, d'abeille (Cera alba), de polyéthylène éventuellement fonctionnalisées, et de paraffine, ainsi que l'ozokérite, les cires microcristallines, les alcools gras linéaires en $C_{14}$-$C_{22}$ et les triesters d'acides en $C_8$-$C_{20}$ et de glycérine tels que le tribéhénate de glycérine, et leurs mélanges, sans que cette liste ne soit limitative. On peut également citer le stéarate de glycol acétylé commercialisé par la société VEVY sous la dénomination commerciale CETACENE®.

Des exemples de gélifiants lipophiles sont notamment les polymères de silicone et plus particulièrement les élastomères d'organopolysiloxanes. Parmi ceux-ci, on peut citer les polymères au moins partiellement réticulés résultant de la réaction d'un organopolysiloxane portant des groupes insaturés, tels que des groupes vinyle ou allyle, situés en bout ou en milieu de chaîne, de préférence sur un atome de silicium, avec un autre composé siliconé réactif tel qu'un organohydrogénopolysiloxane. Ces polymères sont habituellement disponibles sous forme de gel dans un solvant siliconé volatil ou non volatil ou dans un solvant hydrocarboné. Des exemples de tels élastomères sont notamment commercialisés par la société SHIN ETSU sous les dénominations commerciales KSG-6, KSG-16, KSG-31, KSG-32, KSG-41, KSG-42, KSG-43 et KSG-44, et par la société DOW CORNING sous les dénominations commerciales DC 9040 et DC 9041. Un autre gélifiant huileux est constitué d'un polymère de silicone, obtenu par auto-polymérisation d'un organopolysiloxane fonctionnalisé par des groupements epoxy et hydrosilylé, en présence d'un catalyseur, qui est disponible dans le commerce auprès de la société GENERAL ELECTRIC sous la dénomination commerciale VELVESIL® 125. Un autre gélifiant lipophile est constitué d'un copolymère dimethicone / vinyldimethicone cyclique tel que celui commercialisé par la société JEEN sous la dénomination commerciale JEESILC® PS (dont PS-VH, PS-VHLV, PS-CM, PS-CMLV et PS-DM). Un autre type de gélifiants lipophiles est constitué par les copolymères de styrène et d'oléfines telles que l'éthylène, le propylène et/ou le butylène, éventuellement associés à des solvants siliconés ou hydrocarbonés, tels que décrits en particulier dans la demande WO 98/38981 et dans le brevet US-6,309,629. Ils comprennent notamment les gélifiants à base de terpolymères séquencés disponibles auprès de la société PENRECO sous la dénomination commerciale VERSAGEL®. Un autre type de gélifiant lipophile est constitué des polyamides tels que ceux identifiés par le nom INCI polyamide-3 et en particulier les polymères SYLVACLEAR® AF 1900V et PA 1200V disponibles auprès de la société ARIZONA CHEMICAL ainsi que ceux identifiés par le nom INCI « Ethylenediamine/Hydrogenated Dimer Dilinoleate Copolymer Bis-Di-C14-18 Alkyl Amide » et disponibles par exemple sous la dénomination commerciale SYLVACLEAR® A200V ou SYLVACLEAR® A2614V auprès de la société ARIZONA CHEMICAL. Le gélifiant lipophile peut en variante être une bentone ou une hectorite modifiée hydrophobe.

Matières colorantes

La composition selon l'invention peut comprendre, en outre, au moins une matière colorante qui peut être choisie parmi les pigments, les nacres, les colorants liposolubles, les paillettes et leurs mélanges.

Par pigments, il faut comprendre des particules blanches ou colorées, minérales ou organiques, insolubles dans une solution aqueuse, destinées à colorer et/ou opacifier la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être multi-couche, par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730 , EP-A-787731 et WO-A- 96/08537 .

Les pigments utilisés dans la composition cosmétique selon l'invention peuvent être traités en surface par un agent organique.

Les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et/ou avoir subi plusieurs traitements de surface. Ces pigments traités en surface peuvent être préparés selon des techniques de traitement de surface bien connues de l'homme de l'art ou trouvés tels quels dans le commerce.

De préférence, les pigments traités en surface sont recouverts par une couche organique. L'agent organique peut être déposé sur les pigments par évaporation de solvant, réaction chimique entre les molécules de l'agent de surface ou création d'une liaison covalente entre l'agent de surface et les pigments.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

Les pigments peuvent être présents en une teneur allant de 0,01 à 20 % en poids, notamment de 0,1 à 15 % en poids, et en particulier de 1 à 10 % en poids, par rapport au poids total de la composition cosmétique.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en œuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Par "colorants", il faut comprendre des composés généralement organiques solubles dans les corps gras comme les huiles.

Les colorants liposolubles sont par exemple le rouge Soudan, le DC Red 17, le DC Green 6, le $\beta$-carotène, le brun Soudan, le DC Yellow 11, le DC Violet 2, le DC orange 5, le jaune quinoléine.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, "stabilisé" signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et

les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par " dérivés métalliques ", on désigne des composés dérivés de métaux notamment des oxydes, des fluorures, des chlorures et des sulfures.

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO2/Al/SiO2/Al, des pigments ayant cette structure étant commercialisés par la société DUPONT DE NEMOURS ; Cr/MgF2/Al/MgF2/Cr, des pigments ayant cette structure étant commercialisés sous la dénomination CHROMAFLAIR par la société FLEX ; MOS2/SiO2/Al/SiO2/MOS2 ; Fe2O3/SiO2/Al/SiO2/Fe2O3, et Fe2O3/SiO2/Fe2O3/SiO2/Fe2O3, des pigments ayant ces structures étant commercialisés sous la dénomination SICOPEARL par la société BASF ; MoS2/SiO2/mica-oxyde/SiO2MoS2 ; Fe2O3/SiO2/mica-oxyde/SiO2/Fe2O3 ; TiO2/SiO2/TiO2 et TiO2/Al2O3/TiO2 ; SnO/TiO2/SiO2/TiO2/SnO ; Fe2O3/SiO2/Fe2O3; SnO/mica/TiO2/SiO2/TiO2/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe2O3/SiO2/Al/ SiO2/Fe2O3 on passe du doré-vert au gris-rouge pour des couches de SiO2 de 320 à 350 nm ; du rouge au doré pour des couches de SiO2 de 380 à 400 nm ; du violet au vert pour des couches de SiO2 de 410 à 420 nm ; du cuivre au rouge pour des couches de SiO2 de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

Les matières colorantes peuvent être présentes, dans la composition, en une teneur allant de 0,01 % à 50 % en poids, par rapport au poids de la composition, de préférence de 0,1 % à 30 % en poids, et plus préférentiellement de 1 à 20% en poids.

## Phase aqueuse

La composition selon l'invention peut, en outre, comprendre une phase aqueuse comprenant de l'eau et optionnellement un ou plusieurs gélifiants hydrophiles.

La composition selon l'invention peut notamment comprendre une teneur en eau allant de 10 à 50%, de préférence de 15 à 40% en poids, plus préférentiellement de 20 à 30 % en poids, par rapport au poids total de la composition.

Le gélifiant hydrophile peut de préférence être un hydrocolloïde qui peut notamment être choisi parmi : les homo-et copolymères d'acide acrylique et/ou de sels ou d'esters d'acide acrylique, tels que les carbomers, les mélanges à base de polyacrylate de sodium tels que le GELLINOV®, l'HYDRILLIEN 9® de LIPOCHEMICALS et le DC RM 2051® de DOW CORNING ; les homo- et copolymères d'acrylamide ; les homo- et copolymères d'acide acryloylméthylpropane sulfonique (AMPS), tels que l'ARISTOFLEX AVC® ou HMB® de CLARIANT et le SEPINOV EMT® de SEPPIC ; les polymères associatifs tels que l'AdekaNol GT-730 commercialisé par la société Adeka ou le CDS-6000P commercialisé par la société Nihon Surfactant Kogyo K.K., les polysaccharides tels que les gommes de guar ou de xanthane, les carraghénanes, les lévananes greffés et les inulines non greffées ; et les dérivés de cellulose, sans que cette liste ne soit limitative. La phase aqueuse peut également contenir des composés siliconés solubles tels que le mélange commercialisé par la société CLARIANT sous la dénomination commerciale SILCARE® SEA, qui répond au nom INCI « trideceth-9 amodimethicone & trideceth-12 ».

## Tensioactifs

La composition selon l'invention peut également comprendre un ou plusieurs tensioactifs, choisis parmi les émulsionnants eau-dans-huile ou huile-dans-eau,tels que les polysiloxanes modifiés polyéthers, en particulier les polydiméthylsiloxanes oxyéthylénés et/ou oxypropylénés tels que ceux commercialisés par la société DOW CORNING sous la dénomination commerciale 5225C® Formulation Aid et par la société SHIN-ETSU sous la dénomination commerciale KF-6017® ou KF-6028® ou KF-6038®; et le dipolyhydroxystéarate polyéthoxylé (30 OE) commercialisé notamment sous la dénomination commerciale ARLACEL® P135 par la société UNIQEMA.

Actifs

La composition utilisée selon l'invention peut par ailleurs contenir un ou plusieurs actifs hydrophiles ou lipophiles.

La composition selon l'invention peut comprendre au moins un actif pour le blanchiment de la peau capable de bloquer la synthèse de protéines structurales impliquées dans le mécanisme de mélanogenèse (stade I) comme la glycoprotéine mélanocyte-spécifique Pme117. De tels agents peuvent être choisis parmi l'ester cétylique tranexamique (par exemple le trans-4(aminomethyl)cyclohexanecarboxylic acid hexadecyl ester hydrochloride commercialisé par la société Nikko Chemicals) ou l'acide férulique vendu sous le nom commercial Cytovector® (water & Butylene glycol & lectithin & Laurdimonium Hydroxypropyl Hydrolyzed Soy Protein, vendu par la société BASF).

Dans une variante, la composition selon l'invention peut comprendre un agent blanchissant inhibant la synthèse de mélanine, et/ou inhibant l'expression de MITF, et/ou ayant une activité anti-tyrosinase, et/ou inhibant la synthèse de de l'endothelin-1 comme par exemple l'extrait de Licorice (glycyrrhiza glabra extract) vendu par la société Maruzen sous le nom commercial Licorice Extract®.

Dans une autre variante, la composition selon l'invention peut comprendre un agent blanchissant ayant un effet antioxydant, comme les dérivés de Vitamine C, incluant les sels d'ascorbate, les esters d'ascorbate, les esters d'ascorbyl et d'acides gras ou d'acide sorbique, et les autres dérivés d'acide ascorbique comme par exemple les phosphates d'ascorbyl, les esters de saccharide et d'acide ascorbique, comme par exemple l'ascorbyl-2-glucoside, le 2-o-alpha-D-glucopyranosyl L-ascorbate, ou le 6-O- beta -D-galactopyranosyl L-ascorbate. En particulier, un agent de ce type est commercialisé par la société DKSH sous le nom commercial Ascorbyl Glucoside®.

D'autres agents blanchissant pouvant être inclus dans la composition selon l'invention sont des agents dépigmentant de la peau comme par exemple la vitamine B3 (ou Niacinamide), certains extraits de plantes dont l'extrait de Narcissus tazetta, le résorcinol ou rucinol ou leurs dérivés, l'acide glycyrrhizinic et l'hydroquinone-beta-glucoside, la cystéine.

La composition selon l'invention peut également contenir un actif anti-âge qui peut notamment être choisi parmi : les agents stimulant l'expression de la tensine 1; les agents stimulant l'expression de la fructosamine-3-kinase ou de sa protéine apparentée (FN3K RP); les agents stimulant la production de facteurs de croissance; les agents anti-glycation ou déglycants; les agents augmentant la synthèse de collagène ou prévenant sa dégradation (agents anti-collagénases, notamment inhibiteurs de métalloprotéinases matricielles); les agents augmentant la synthèse d'élastine ou prévenant sa dégradation (agents anti-élastases); les agents augmentant la synthèse de glycosaminoglycanes ou de protéoglycanes ou prévenant leur dégradation (agents anti-protéoglycanases); les agents stimulant la synthèse d'intégrines par les fibroblastes ; les agents augmentant la prolifération ou la différenciation des kératinocytes; les agents augmentant la prolifération des fibloblastes; les agents anti-oxydants ou anti-radicalaires ou anti-pollution; et leurs mélanges, sans que cette liste ne soit limitative.

Des exemples de tels agents sont notamment : les extraits de plantes et en particulier les extraits de *Butea frondosa,* de *Magnolia champaca,* de *Vanilla planifolia,* de *Cedrus atlantica,* de *Canarium commune* (elemi), de *Zingiber cassumunar Roxb.,* de *Chondrus crispus,* de *Thermus thermophilus,* de *Pisum sativum,* de *Centella asiatica,* de Scenedesmus, de *Moringa pterygosperma,* d'hamamélis, de *Castanea sativa, d'Hibiscus sabdriffa,* de *Polyanthes tuberosa, d'Argania spinosa, d'Aloe vera,* de *Narcissus tarzetta,* ou de réglisse; une huile essentielle de *Citrus aurantium* (Neroli); les $\alpha$-hydroxyacides tels que les acides glycolique, lactique et citrique, et leurs esters; les $\beta$-hydroxyacides, tels que l'acide salicylique et ses dérivés; les hydrolysats de protéines végétales (notamment de soja ou de noisette); les oligopeptides acylés; les extraits de levure et en particulier de *Saccharomyces cerevisiae;* les extraits d'algues et en particulier de laminaires; les vitamines et leurs dérivés tels que le palmitate de rétinyle, l'acide ascorbique, le glucoside d'ascorbyle, l'ascorbyl phosphate de magnésium ou de sodium, le palmitate d'ascorbyle, le tétraisopalmitate d'ascorbyle, le sorbate d'ascorbyle, le tocophérol, l'acétate de tocophéryle et le sorbate de tocophéryle; et leurs mélanges.

La composition utilisée selon l'invention peut en outre renfermer des agents humectants tels que l'acide hyaluronique et ses sels et/ou les polyols tels que la glycérine.

De préférence, la composition selon l'invention contient un actif blanchissant de la peau choisi parmi la licorice, la niacinamide, l'ascorbyl glucoside et leurs mélanges.

Ingrédients cosmétiques usuels additionnels

La composition selon l'invention peut également comprendre tout ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

En particulier, la composition selon l'invention peut comprendre au moins un filtre solaire, qui peut être un filtre organique ou minéral ou un mélange des deux.

A titre d'illustration des filtres UV organiques et de façon non limitative, on peut citer:

- les anthranilates, en particulier l'anthranilate de menthyle ;
- les benzophénones, en particulier la benzophénone-1, la benzophénone-3 ou oxybenzone, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40® disponible chez B.A.S.F.) ;
- les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène dicamphre sulfonique, et préférentiellement le 4-méthylbenzylidène camphre (Eusolex 6300® disponible chez Merck) ;
- les benzimidazoles, en particulier le benzimidazilate (Neo Heliopan AP® disponible chez Haarmann et Reimer), ou l'acide phénylbenzimidazole sulfonique (PARSOL HS® disponible chez DSM) ;
- les benzotriazoles, en particulier le drométrizole trisiloxane, ou le méthylène bis-benzotriazolyltétraméthylbutylphénol (Tinosorb M® disponible chez Ciba) ;
- les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, Kaempferia galanga root extract (TEGO GALANGA d'EVONIK contenant 98% ethyl-p-methoxycinnamate) et préférentiellement l'octylméthoxycinnamate (Parsol MCX® disponible chez Hoffmann La Roche),
- les diphenylacrylates en particulier l'éthocrylène (Uvinul N35® disponible chez B.A.S.F.), ou l'octocrylène (Uvinul 539® disponible chez B.A.S.F.) ou Ethylhexyl methoxycrylene (SOLASTAY disponible chez HALLSTAR)
- les dibenzoylméthanes, en particulier le butyl méthoxydibenzoylméthane (Parsol 1789®) ; les imidazolines, en particulier l'éthylhexyl diméthoxybenzylidène dioxoimidazoline;
- les PABA, en particulier l'éthyl Dihydroxypropyl PABA, l'éthylhexyldiméthyl PABA, le glycéryl PABA, le PABA, le PEG-25 PABA, ou l'éthyl PABA (benzocaïne),
- les triazines, en particulier l'anisotriazine (Tinosorb S® disponible chez Ciba) ou la diéthylhexylbutamido-triazone (Uvasorb HEB® disponible chez 3V Sigma), l'éthylhexyltriazone (Uvinul T150® disponible chez B.A.S.F.), le Tris-Biphenyl Triazine (Tinosorb 2AB disponible chez BASF),
- les benzoates, en particulier le N-hexyl 2-(4-diethylamino-2-hydroxybenzoyl)benzoate (Uvinul A+ disponible chez BASF) ou en mélange avec octyl methoxycinnamate (Uvinul A+B disponible chez BASF),
- les benzalmalonates, en particulier le Polysilicone-15 (Parsol SLX disponible chez DSM),
- les benzoxazoles en particulier le 2,4-Bis[4-[5-(1,1-dimethylpropyl)benzoxazol-2-yl]phenylimino]-6-[(2-ethylhexyl)imino]- 1,3,5-triazine, (Uvasorb K2A disponible chez Sigma 3V),
- les salicylates, en particulier le salicyclate de dipropylèneglycol, le salicylate d'éthylhexyle, l'homosalate, le butyloctyl Salicylate (HALLBRITE BHB disponible chez HALLSTAR ou le TEA salicylate ;

Les filtres organiques préférés sont choisis parmi Phenylbenzimidazole sulfonic acid (Parsol HS), Octocrylene (Parsol 340), Ethylhexyl Methoxycinnamate (Parsol MCX), Methylene Bis-Benzotriazolyl Tetramethylbutylphenol (Tinosorb M), Bis-Ethylhexyloxyphenol Methoxyphenyl Triazine (Tinosorb S), Tris-Biphenyl Triazine (Tinosorb 2AB),Ethylhexyl Triazone (UVINUL T150), Diethylhexyl Butamido Triazone (UVASORB HEB), Diethylamino Hydroxybenzoyl Hexyl Benzoate (UVINUL A PLUS), Butyl Methoxydibenzoylmethane (PARSOL 1789), Polysilicone-15 (PARSOL SLX), Benzophénone-4 (Uvinul MS 40), Benzophenone-3 Ethylhexyl Salicylate (Parsol EHS), Homosalate et leurs mélanges.

Les filtres UV organiques sont préférablement présents dans les compositions de l'invention de 0.1% à 40% en poids et en particulier de 5 à 25 %

Les filtres UV inorganiques utilisés sont des particules d'oxyde métallique ayant une taille moyenne de particule élémentaire inférieure ou égale à 100 nm.

Ils peuvent être notamment choisis parmi les oxydes de titane, de zinc, de fer, de zirconium, de cérium ou leurs mélanges.

Les oxydes de titane peuvent se présenter sous une forme cristallisée de type rutile et/ou anatase, et/ou sous une forme amorphe ou substantiellement amorphe.

De tels pigments d'oxydes métalliques, enrobés ou non enrobés sont en particulier décrits dans la demande de brevet EP-A-0518 773. A titre de pigments commerciaux on peut mentionner les produits vendus les sociétés Kemira, Tayca, Merck et Degussa.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments d'oxydes de titane enrobés sont enrobés:

- de silice (SUNVEIL d'IKEDA),
- de silice et d'oxyde de fer (SUNVEIL F d'IKEDA),
- de silice et de polyglyceryl-10 stearate (COSMESERVE WP-40W de IWASE COSFA)
- de silice et d'alumine (MICROTITANIUM DIOXIDE MT500 SA et MICROTITANIUM DIOXIDE MT 100 SA de TAYCA, TIOVEIL de TIOXIDE),
- d'alumine (TIPAQUE TTO-55 (B) et TIPAQUE TTO-55 (A) d'ISHIHARA, et UVT 14/4 de KEMIRA),
- de TiO2 rutile traité avec l'alumine et de la silice enrobé de glycérol (UV TITAN M212 de KEMIRA),
- de TiO2 rutile traité avec l'alumine et de la diméthicone (UV TITAN M195 de KEMIRA),
- d'alumine et de stéarate d'aluminium (MICROTITANIUM DIOXIDE MT 100 T, MT 100 TV, MT 100 TX, MT 100 Z, MT-01 de TAYCA, Solaveil CT-10 W et Solaveil CT 100 de UNIQEMA et Eusolex T-AVO de MERCK),
- de silice, d'alumine et d'acide alginique (MT-100 AQ de TAYCA),
- d'alumine et de laurate d'aluminium (MICROTITANIUM DIOXIDE MT 100 S de TAYCA),
- d'alumine, de methicone et d'acide polyhydroxystearique (INP60T7 de KOBO)
- d'oxyde de fer et de stéarate de fer (MICROTITANIUM DIOXIDE MT 100 F de TAYCA),
- d'oxyde de zinc et de stéarate de zinc (BR 351 de TAYCA),
- de silice et d'alumine et traités par une silicone (MICROTITANIUM DIOXIDE MT 600 SAS, MICROTITANIUM DIOXIDE MT 500 SAS ou MICROTITANIUM DIOXIDE MT 100 SAS de TAYCA),
- de silice, d'alumine, de stéarate d'aluminium et traités par une silicone (STT-30-DS de TITAN KOGYO),
- d'alumine et traités par une silicone (TIPAQUE TTO-55 (S) de ISHIHARA, ou UV TITAN M 262 de KEMIRA),
- de triéthanolamine (STT-65-S de TITAN KOGYO),
- d'acide stéarique (TIPAQUE TTO-55 (C) de ISHIHARA,
- d'hexamétaphosphate de sodium (MICROTITANIUM DIOXIDE MT 150 W de TAYCA).
- le TiO2 traité par l'octyl triméthyl silane (T 805 par la société DEGUSSA SILICES),
- le TiO2 traité par un polydiméthylsiloxane (70250 Cardre UF TiO2S13 par CARDRE),
- le TiO2 anatase/rutile traité par un polydiméthylhydrogénosiloxane (MICRO TITANIUM DIOXYDE USP GRADE HYDROPHOBIC par COLOR TECHNIQUES).
- le TiO2 rutile traité par l'alumine, l'acide stéarique (UV TITAN M160 par KEMIRA) .
- le TiO2 traité par l'alumine hydroxyde, l'acide stéarique et le triethoxycaprylylsilane (ALT-T-400 par MAPRECOS ou Titanium Dioxide & Aluminium hydroxide & Stearic acid (ST-705SA /TITAN KOGYO))
- le TiO2 dopé au manganese (OPT1-PW de Croda)

Les pigments d'oxyde de titane non enrobés sont par exemple :

- MICROTITANIUM DIOXIDE MT 500 B ou MICROTITANIUM DIOXIDE MT600 B par la société TAYCA,
- P 25 par la société DEGUSSA,
- Oxyde de titane transparent PW par la société WACKHER,
- UFTR par la société MIYOSHI KASEI,
- ITS par la société TOMEN

et TIOVEIL AQ par la société TIOXIDE.

Les pigments d'oxyde de zinc non enrobés, sont par exemple:

- Z-cote par la société Sunsmart;
- Nanox par la société Elementis;
- Nanogard WCD 2025 par la société Nanophase Technologies;

Les pigments d'oxyde de zinc enrobés sont par:

- Oxide zinc CS-5 par la société Toshibi (ZnO enrobé par polymethylhydrogenesiloxane) ;
- Nanogard Zinc Oxide FN par la société Nanophase Technologies (en dispersion à 40% dans le Finsolv TN, benzoate d'alcools en C12-C15);

- DAITOPERSION ZN-30 et DAITOPERSION Zn-50 par la société Daito (dispersions dans cyclopolyméthylsiloxane /polydiméthylsiloxane oxyéthyléné, contenant 30% ou 50% de nano-oxydes de zinc enrobés par la silice et le s polyméthylhydrogènesiloxane) ;
- NFD Ultrafine ZnO par la société Daikin (ZnO enrobé par phosphate de perfluoroalkyle et copolymère à base de perfluoroalkyléthyle en dispersion dans du cyclopentasiloxane) ;
- SPD-Z1 par la société Shin-Etsu (ZnO enrobé par polymère acrylique greffé silicone, dispersé dans cyclodiméthylsiloxane) ;
- Escalol Z100 par la société ISP (ZnO traité alumine et dispersé dans le mélange methoxycinnamate d'ethylhexyle / copolymère PVP-hexadecene / methicone);
- Fuji ZnO-SMS-10 par la société Fuji Pigment (ZnO enrobé silice et polymethylsilsesquioxane);
- Nanox Gel TN par la société Elementis (ZnO dispersé à 55% dans du benzoate d'alcools en C12-C15 avec polycondensat d'acide hydroxystéarique).
- OTS-5 MZ-500 par la société DAITO (ZnO dispersé dans triethoxycaprylylsilane).

Les pigments d'oxyde de cérium non enrobés peuvent être par exemple ceux vendus sous la dénomination COLLOIDAL CERIUM OXIDE par la société RHONE POULENC.

Selon l'invention, les pigments d'oxyde de titane ou d'oxyde de zinc, enrobés ou non enrobés, sont particulièrement préférés.

Les filtres inorganiques sont de préférence présents dans les compositions selon l'invention à une teneur allant de 0,1 % à 40 % en poids et en particulier de 5 à 25 % en poids, par rapport au poids total de la composition.

La composition selon l'invention peut également comprendre un oxyde de titane pigmentaire particulier enrobé par de l'hydroxyde d'aluminium et de l'acide stéarique commercialisé par la société Titan Kogyo sous le nom ST 705 SA. Ce pigment présente une forte transmittance des rayons de couleur rouge tout en bloquant les autres rayons du spectre visible, et possède un effet booster du SPF.

Les exemples suivants sont donnés à titre illustratif et non limitatif de la présente invention. Les pourcentages sont des pourcentages en poids.

**Exemple 1:**

On a préparé une poudre compacte selon l'invention ayant la composition suivante :

| Nom INCI (Nom commercial / Fournisseur) | Fonction | % en poids |
|---|---|---|
| **Phase Pulvérulente** | | |
| Synthetic Fluorphlogopite (PDM-5L /TOPY INDUSTRIES) | Agent de texture et de confort thermique | qsp |
| Mica (MICA 1000 /SCIAMA) | Agent de texture et de confort thermique | 5 |
| Boron Nitride (SOFTOUCH CC6059 BN /MOMENTIVE) | Agent de texture et de confort thermique | 8 |
| Silica (SPHERICA P 1000 /IKEDA) | Agent de texture et de confort thermique | 5 |
| Vinyl Dimethicone/Methicone Silsesquioxane Crosspolymer (KSP-100 /SHIN ETSU) | Absorption sébum et Confort thermique | 6 |
| Nylon-12 (ORGASOL 2002 EXD NAT COS /ARKEMA) | Agent de texture et de confort thermique | 2 |
| Polymethylsilsesquioxane (KMP-590 /SHIN ETSU) | Agent de texture et de confort thermique | 3 |
| Mica & Titanium Dioxide (PRESTIGE SUPER SOFT SILVER /SURDASHAN CHEMICAL INDUSTRIES) | Nacre | 1 |
| Titanium dioxide & Perfluorooctyl triethoxysilane & aluminium hydroxide (FHS UFTI 40A /DAITO KASEI) | Filtre UV minéral | 15 |

(suite)

| Nom INCI (Nom commercial / Fournisseur) | Fonction | % en poids |
|---|---|---|
| **Phase Pulvérulente** | | |
| Silica & Titanium dioxide & sodium potassium aluminium silicate (RONAFLAIR LDP WHITE /MERCK) | Confort thermique | 2,5 |
| Titanium Dioxide & Aluminium hydroxide & Stearic acid (ST-705SA /TITAN KOGYO) | Filtre UV mineral | 1 |
| Pigments traités hydrophobes (traitement de surface: Perfluorooctyl triethoxysilane & aluminium hydroxide) | Pigment | 5 - 10 |
| Actifs (uniformité du teint) | actif | 0,1-0,5 |
| Additifs de compactage | additif | 0,5 |
| **Phase grasse** | | |
| Ethylhexyl Methoxycinnamate (Parsol MCX / DSM) | Filtre UV organique | 7,5 |
| Dimethicone (Mirasil DM100 / Bluestar) | Liant gras | 2 |
| Sorbitan sesquiisostearate (Salacos 182V) | Stabilisant | 0,1 |

Les ingrédients de la phase pulvérulente sont pesés et mélangés deux fois 2 minutes au Blixer. Puis la composition est préparée selon le protocole suivant :

- ajouter la phase grasse et mélanger trois fois 2 minutes au Blixer,
- broyer le mélange obtenu.
- tamiser puis compacter la poudre.

La poudre compacte obtenue a été appliquée sur la moitié du visage, sur un panel de 29 femmes (versus peau nue sur l'autre moitié du visage). La moitié de ces femmes présentant une peau à tendance mixte/grasse avec des pores visibles, a été placée dans des conditions chaudes et humides (T: 30°C; HR: 75%). L'autre moitié présentant une peau à tendance mixte/sèche et ayant déclaré une sensibilité au froid (apparence de plaques rouges, démangeaisons et tiraillements), a été placée dans des conditions froides et sèches (T: 7°C; HR: 4 0%).

Résultats de l'évaluation dans les conditions Chaudes et Humides (T: 30°C; HR: 75%):

Les figures 1 et 2 illustrent par des diagrammes l'évaluation de la visibilité des pores de la peau et du confort ressenti par l'utilisatrice dans des conditions chaudes et humides. Sur la figure 2, l'évaluation à T0 correspond à la peau nue (avant application de la poudre compacte) et l'évaluation Tia correspond à l'évaluation réalisée immédiatement après application de la poudre compacte.

Résultats de l'évaluation dans les conditions froides et sèches (T: 7°C; HR: 40%):

Les figures 3 et 4 illustrent par des diagrammes l'évaluation de la visibilité des rougeurs de la peau et du confort ressenti par l'utilisatrice dans des conditions froides et sèches.
Les résultats ont montré que la composition comprenant les charges de faible conductivité permettait de diminuer la visibilité des pores lorsqu'il fait chaud et humide (Figure 1), de diminuer la visibilité des rougeurs lorsqu'il fait froid et sec (Figure 3), et d'améliorer le confort de la peau dans les deux cas (Figures 2 et 4). Sur la figure 4, To et Tia ont la même signification que sur la figure 2.

**Exemple 2:**

Nous avons préparé des émulsions huile-dans-eau selon l'invention, ayant l'une des compositions suivantes. Les compositions 2-a à 2-f sont formulées avec des pigments hydrophobes dans la phase grasse interne, et les compositions 2-g et 2-h sont formulées avec des pigments hydrophiles dans la phase aqueuse externe.

| | Ex 2-a | Ex 2-b | Ex 2-c | Ex 2-d | Ex 2-e | Ex 2-f | Ex 2-g | Ex 2-h |
|---|---|---|---|---|---|---|---|---|
| **Nom INCI (Nom commercial-Fournisseur)** | **% en poids** | | | | | | | |
| **Pigments** | | | | | | | | |
| Pigments traités Amino-acide (Iron oxides and Titanium Dioxide) (pigments traités NAI de Miyoshi Kaseï, ou pigments traités LL de Daïto, ou pigments traités ASL ou ASI de Daïto) | 7,5 | - | - | - | - | - | - | - |
| Pigments traités Fluoro (Iron oxides and Titanium Dioxide) (pigments traités FHS de Daïto Kaseï ou pigments traités FHP de Sensient) | - | 7,5 | - | - | - | - | - | - |
| Pigments traités Lécithine (Iron oxides and Titanium Dioxide) (pigments traités HLC de Sensient) | - | - | 7,5 | - | - | - | - | - |
| Pigments traités Alkylsilane (Iron oxides and Titanium Dioxide) (pigments traités OTS de Daïto) | | - | - | 7,5 | - | - | - | - |
| Pigments traités résine de silicone (Trimethylsiloxysilicate & Polymethylsilsesquioxane-Pigments traités SR de Gelest) | - | - | - | - | 7,5 | - | - | - |
| Timica Terra (Iron Oxides and Titanium Dioxide-BASF) | - | - | - | - | - | 7,5 | - | - |
| Titanium Dioxide & Stearic acid & Alumina | 4 | 4 | 4 | 4 | 4 | 4 | 4 | 4 |
| Iron oxide & silica (Sympholite-Presperse Inc) | - | - | - | - | - | - | 7,5 | - |
| PEG-12 Dimethicone (série SGP de Sensient) | - | - | >- | - | - | - | - | 7,5 |
| **Charges** | | | | | | | | |
| Talc & Dimethicone | 1,6 | 1,6 | 1,6 | 1,6 | 1,6 | 1,6 | 1,6 | 1,6 |
| SILICA & TITANIUM DIOXIDE & SODIUM POTASSIUM ALUMINUM SILICATE (Ronaflair LDP White-Merck) | 1,5 | 1,5 | 1,5 | 1,5 | 1,5 | 1,5 | 1,5 | 1,5 |
| BORON NITRIDE (Softouch CC6059 BN-Momentive) | 1,5 | 1,5 | 1,5 | 1,5 | 1,5 | 1,5 | 1,5 | 1,5 |
| **Phase grasse** | | | | | | | | |
| Caprylyl methicone | 5,5 | 5,5 | 5,5 | 5,5 | 5,5 | 5,5 | 5,5 | 5,5 |
| PVP /eicosene copolymer (Antaron V220-Ashland Industries) | 0,5 | 0,5 | 0,5 | 0,5 | 0,5 | 0,5 | 0,5 | 0,5 |
| Cetyl Ethylhexanoate (Trivent OC-16-Alzo) | 2 | 2 | 2 | 2 | 2 | 2 | 2 | 2 |
| Ethylhexyl Methoxycinnamate | 7,5 | 7,5 | 7,5 | 7,5 | 7,5 | 7,5 | 7,5 | 7,5 |
| Sorbitan Tristearate | 0,2 | 0,2 | 0,2 | 0,2 | 0,2 | 0,2 | 0,2 | 0,2 |
| **Phase Aqueuse** | | | | | | | | |
| DIMETHICONE & AQUA (WATER) & GLYCERIN & PENTYLENE GLYCOL & DIMETHICONE/VINYL DIMETHICONE CROSSPOLYMER & AMODIMETHICONE & PHENOXYETHANOL & CARBOMER & SODIUM HYDROXIDE & DISODIUM EDTA (CES-1104-Nusil) | 10 | 10 | 10 | 10 | 10 | 10 | 10 | 10 |

(suite)

| Phase Aqueuse | | | | | | | | | |
|---|---|---|---|---|---|---|---|---|
| TRIFLUOROPROPYL DIMETHICONE & AQUA (WATER) & GLYCERIN & PENTYLENE GLYCOL & AMODIMETHICONE & CARBOMER & SODIUM HYDROXIDE & PHENOXYETHANOL & DISODIUM EDTA (CES-3401-Nusil) | 10 | 10 | 10 | 10 | 10 | 10 | 10 | 10 |
| Hydroxyethylacrylate / sodium acruylloyldimethyl Taurate copolymer & Sorbitan Isostearate & Polysorbate 60 (Sepinov EMT 10 - Seppic) | 0,5 | 0,5 | 0,5 | 0,5 | 0,5 | 0,5 | 0,5 | 0,5 |
| Xanthan gum | 0,2 | 0,2 | 0,2 | 0,2 | 0,2 | 0,2 | 0,2 | 0,2 |
| Glycerine | 3,5 | 3,5 | 3,5 | 3,5 | 3,5 | 3,5 | 3,5 | 3,5 |
| Butylene glycol | 1,5 | 1,5 | 1,5 | 1,5 | 1,5 | 1,5 | 1,5 | 1,5 |
| Caprylyl glycol | 0,4 | 0,4 | 0,4 | 0,4 | 0,4 | 0,4 | 0,4 | 0,4 |
| Phenoxyethanol | 0,6 | 0,6 | 0,6 | 0,6 | 0,6 | 0,6 | 0,6 | 0,6 |
| Alcohol | 2 | 2 | 2 | 2 | 2 | 2 | 2 | 2 |
| Silica (Spheron P1000-Presperse) | 2 | 2 | 2 | 2 | 2 | 2 | 2 | 2 |
| Water | qsp | qsp | qsp | qsp | qsp | qsp | qsp | qsp |
| **total** | **100** | **100** | **100** | **100** | **100** | **100** | **100** | **100** |

Le mode opératoire mis en œuvre pour la préparation des exemples 2-a à 2-f est le suivant :

- homogénéiser la phase grasse au rotor stator à 75°C,
- broyer les pigments traités au broyeur tricylindre dans le caprylyl méthicone et le cetyl ethylhexanoate,
- ajouter les pigments traités au reste de la phase grasse, sous agitation au rotor stator pendant 10 minutes à 2500 tours par minute,
- mélanger le nitrure de bore et la silice enrobée avec les polyols, puis ajouter les conservateurs et l'eau, puis le gélifiant de la phase aqueuse.
- ajouter l'émulsionnant et les charges, et mélanger à 80°C.
- ajouter les CES-1104 et CES-3401 et mélanger à 80°C,
- ajouter lentement la phase grasse à la phase aqueuse, et mélanger complètement pour former l'émulsion huile-dans-eau,
- refroidir ensuite jusqu'à 30°C et ajouter l'alcool à faible cisaillement,
- enfin, démousser la composition obtenue.

Le mode opératoire mis en œuvre pour la préparation des exemples 2-g et 2-h est le suivant :

- homogénéiser la phase grasse au rotor stator à 75°C,
- broyer les pigments traités au broyeur tricylindre dans la glycérine et/ou le butylène glycol,
- mélanger le nitrure de bore et la silice enrobée avec les polyols, puis ajouter les conservateurs et l'eau, puis le gélifiant de la phase aqueuse,
- ajouter ensuite les pigments traités, l'émulsionnant et la charge, et les mélanger à 80°C,
- ajouter les CES-1104 et CES-3401 et mélanger à 80°C,
- ajouter lentement la phase grasse à la phase aqueuse, et mélanger complètement pour former l'émulsion huile-dans-eau,
- refroidir ensuite jusqu'à 30°C, et ajouter l'alcool à faible cisaillement,
- enfin, démousser la composition obtenue.

**Exemple 3:**

Nous avons préparé des émulsions huile-dans-eau selon l'invention, ayant l'une des compositions suivantes.

Les compositions 3-a à 3-f sont formulées avec des pigments hydrophobes dans la phase grasse interne, et les compositions 3-g et 3-h sont formulées avec des pigments hydrophiles dans la phase aqueuse externe.

| | Ex 3-a | EX 3-b | Ex 3-c | Ex 3-d | Ex 3-e | Ex 3-f | Ex 3-g | Ex 3-h |
|---|---|---|---|---|---|---|---|---|
| **Nom INCI-Nom commercial-Fournisseur** | % en poids | | | | | | | |
| **Phase pulvérulente** | | | | | | | | |
| Pigments traités Amino-acide (Iron oxides and Titanium Dioxide) (pigments traités NAI de Miyoshi Kaseï, ou pigments traités LL de Daïto, ou pigments traités ASL ou ASI de Daïto) | 7,7 | - | - | - | - | - | - | - |
| Pigments traités Fluoro (Iron oxides and Titanium Dioxide) (pigments traités FHS de Daïto Kaseï ou pigments traités FHP de Sensient) | - | 7,7 | - | - | - | - | - | - |
| Pigments traités Lécithine (Iron oxides and Titanium Dioxide) (pigments traités HLC de Sensient) | - | - | 7,7 | - | - | - | - | - |
| Pigments traités Alkylsilane (Iron oxides and Titanium Dioxide) (pigments traités OTS de Daïto) | - | - | - | 7,7 | - | - | - | - |
| Pigments traités résine de silicone (Trimethylsiloxysilicate & Polymethylsilsesquioxane-Pigments traités SR de Gelest) | - | - | - | - | 7,7 | - | - | - |
| Timica Terra Iron Oxides and pigmentary Titanium Dioxide de BASF | - | - | - | - | - | 7,7 | - | - |
| Iron oxide & silica (Sympholite de Presperse Inc) | - | - | - | - | - | - | 7,7 | - |
| PEG-12 Dimethicone (SGP de Sensient) | - | - | - | - | - | - | - | 7,7 |
| Nano Titanium Dioxide (Traitement de surface: acide stéarique ou alkylsilane) | 4 | 4 | 4 | 4 | 4 | 4 | 4 | 4 |
| Nano Zinc Oxide (Traitement de surface: acide stéarique ou alkylsilane) | 4 | 4 | 4 | 4 | 4 | 4 | 4 | 4 |
| Boron Nitride (TRES BN PUHP3002 - Saint-Gobain Advanced Ceramics) | **3** | **3** | **3** | **3** | **3** | **3** | **3** | **3** |
| SILICA & TITANIUM DIOXIDE & SODIUM POTASSIUM ALUMINUM SILICATE (Ronaflair LDP White de Merck) | **1,5** | **1,5** | **1,5** | **1,5** | **1,5** | **1,5** | **1,5** | **1,5** |
| **Phase grasse** | | | | | | | | |
| Methyl Trimethicone | 7 | 7 | 7 | 7 | 7 | 7 | 7 | 7 |
| Dimethicone 5 cSt | 6 | 6 | 6 | 6 | 6 | 6 | 6 | 6 |
| Ethylhexyl methoxycinnamate | 5 | 5 | 5 | 5 | 5 | 5 | 5 | 5 |
| Caprylic / Capric Triglycéride | 3,7 5 | 3,7 5 | 3,7 5 | 3,7 5 | 3,7 5 | 3,7 5 | 3,7 5 | 3,7 5 |
| Dimethicone & trimethylsiloxysilicate/dimet hiconol crosspolymer (DC 7-4411 Cosmetic Fluid-Dow Corning) | 3 | 3 | 3 | 3 | 3 | 3 | 3 | 3 |
| **Phase Aqueuse** | | | | | | | | |
| CES-1104 (Nusil) | 10 | 10 | 10 | 10 | 10 | 10 | 10 | 10 |
| Water | Qsp | Qsp | Qsp | Qsp | Qsp | Qsp | Qsp | Qsp |
| Xanthan gum | 0,1 2 | 0,1 2 | 0,1 2 | 0,1 2 | 0,1 2 | 0,1 2 | 0,1 2 | 0,1 2 |

(suite)

| Phase Aqueuse | | | | | | | | |
|---|---|---|---|---|---|---|---|---|
| Polyols Butylene Glycol and propylene Glycol | 8 | 8 | 8 | 8 | 8 | 8 | 8 | 8 |
| Phospholipid (Emulmetik 320-L.M. Cosmetics | 0,5 | 0,5 | 0,5 | 0,5 | 0,5 | 0,5 | 0,5 | 0,5 |
| Diglycerin | 1,5 | 1,5 | 1,5 | 1,5 | 1,5 | 1,5 | 1,5 | 1,5 |
| Phenoxyethanol | 0.7 5 | 0.7 5 | 0.7 5 | 0.7 5 | 0.7 5 | 0.7 5 | 0.7 5 | 0.7 5 |
| **Total** | **100** | **100** | **100** | **100** | **100** | **100** | **100** | **100** |

Le mode opératoire mis en œuvre pour la préparation des compositions 3-a à 3- f est le suivant :

- broyer le nitrure de bore dans le propylène glycol à l'aide du tricylindre,
- Mélanger tous les ingrédients de la phase aqueuse sauf les CES-1104 et/ou les CES-3401 à environ 75°C environ 20 minutes pour permettre au phospholipide de s'hydrater,
- broyer les pigments dans la dimethicone utilisant le tricylindre,
- ajouter les autres ingrédients de la phase grasse, les filtres UV et la poudre soft focus aux pigments broyés sous fort cisaillement pendant environ 10 minutes à 75°C,
- mélanger jusqu'à obtention d'un mélange homogène,
- ajouter lentement la phase grasse à la phase aqueuse à 75°C sous un cisaillement élevé et laisser mélanger pendant environ 10 minutes,
- refroidir sous cisaillement élevé jusqu'à température ambiante,
- ajouter les CES-1104 et/ou CES-3401 à l'émulsion,
- mélanger à nouveau et homogénéiser à température ambiante.

Le mode opératoire mis en œuvre pour la préparation des compositions 3-g et 3- f est le suivant :

- broyer le nitrure de bore et des pigments dans le propylene glycol et le butylène glycol à l'aide d'un tricylindre,
- mélanger tous les ingrédients de la phase aqueuse sauf les CES-1104 et/ou CES-3401 à environ 75°C pendant 20 minutes: afin de permettre au phospholipide de s'hydrater,
- mélanger tous les ingrédients de la phase grasse, les filtres UV et la poudre soft focus sous cisaillement élevé pendant environ 10 minutes à 75°C,
- mélanger jusqu'à obtention d'un mélange homogène,
- ajouter lentement la phase grasse à la phase aqueuse à 75°C sous un cisaillement élevé et laisser mélanger pendant environ 10 minutes,
- refroidir sous cisaillement élevé jusqu'à température ambiante,
- ajouter les CES-1104 et/ou CES-3401 à l'émulsion. Mélanger à nouveau et homogénéiser à la température ambiante.

## Revendications

1. Utilisation d'au moins une poudre présentant une conductivité thermique inférieure ou égale à 2 W.m$^{-1}$.K$^{-1}$, de préférence comprise entre 0 et 2 W.m$^{-1}$.K$^{-1}$, plus préférentiellement entre 0,1 et 1,5 W.m$^{-1}$.K$^{-1}$ pour améliorer le confort thermique conféré par une composition cosmétique de maquillage ou de soin de la peau.

2. Utilisation selon la revendication 1, **caractérisée en ce que** l'amélioration du confort thermique est obtenue en diminuant la visibilité des pores dans des conditions représentatives d'une chaleur humide (par exemple 30°C, et 75% d'humidité relative), ou en diminuant les rougeurs dans les conditions d'un froid sec (par exemple 7°C, 40% d'humidité relative).

3. Utilisation selon l'une quelconque des revendications précédentes, **caractérisée en ce que** la poudre est une particule de forme sphérique ou lamellaire, minérale ou organique, insoluble dans la composition cosmétique.

4. Utilisation selon l'une quelconque des revendications précédentes, **caractérisée en ce que** la poudre est choisie parmi le talc, la silice, l'alumine, les silicates d'aluminium, notamment les borosilicates d'aluminium comme les billes de verre, le silicate de magnésium et d'aluminium comme le fluorphlogopite, la bentonite, les microsphères de

céramique enrobées de $TiO_2$ et de $SiO_2$, le phosphate de calcium comme l'hydroxyapatite, le sulfate de magnésium, le carbonate de magnésium, le kaolin, le nitrure de bore, la cellulose, le mica, les poudres de PMMA, les polyamides, les résines de silicone telles que les polyméthylsilsesquioxanes, les poudres d'élastomère de silicone éventuellement enrobées par une résine de silicone, et leurs mélanges.

5. Utilisation selon l'une quelconque des revendications précédentes, **caractérisée en ce que** la poudre est choisie parmi le nitrure de bore, le talc, le mica, les microsphères de céramique enrobées de $TiO_2$ et de $SiO_2$, les billes de verre, la porcelaine, et leurs mélanges.

6. Utilisation selon l'une quelconque des revendications précédentes, **caractérisée en ce que** la poudre est choisie parmi le nitrure de bore, les microsphères de céramique enrobées de $TiO_2$ et de $SiO_2$, et leurs mélanges.

7. Utilisation selon l'une quelconque des revendications précédentes, **caractérisée** en ce la composition cosmétique comprenant la poudre de conductivité thermique inférieure ou égale à $2 \text{ W.m}^{-1} \text{ .K}^{-1}$ se présente sous la forme d'une poudre, d'une émulsion, d'une microémulsion, d'une nanoémulsion, d'une suspension, d'une solution, d'une lotion, d'une crème, d'un gel aqueux ou hydroalcoolique, d'une mousse, d'un sérum, d'une composition anhydre ou d'une dispersion pour aérosol.

8. Utilisation selon l'une quelconque des revendications précédentes, **caractérisée en ce que** la composition cosmétique comprenant la poudre de conductivité thermique inférieure ou égale à $2 \text{ W.m}^{-1}.\text{K}^{-1}$ se présente sous forme d'une composition fluide.

9. Utilisation selon l'une quelconque des revendications précédentes, **caractérisée en ce que** la composition cosmétique comprenant la poudre de conductivité thermique inférieure ou égale à $2 \text{ W.m}^{-1}.\text{K}^{-1}$ se présente sous forme d'une poudre libre, compacte ou coulée, de préférence d'une poudre compacte.

10. Utilisation selon la revendication 8, **caractérisée en ce que** la poudre de conductivité thermique inférieure ou égale à $2 \text{ W.m}^{-1} \text{ .K}^{-1}$ représente 0,1 à 50% en poids, de préférence 1 à 20% en poids, et plus préférentiellement 2 à 15% en poids, par rapport au poids total de la composition cosmétique la comprenant.

11. Utilisation selon la revendication 9, **caractérisée en ce que** la poudre de conductivité thermique inférieure ou égale à $2 \text{ W.m}^{-1} \text{ .K}^{-1}$ représente 10 à 90% en poids, de préférence 20 à 80% en poids, et plus préférentiellement 35 à 70% en poids, par rapport au poids total de la composition cosmétique la comprenant.

12. Utilisation selon l'une quelconque des revendications précédentes, **caractérisée en ce que** la composition cosmétique comprend au moins une charge additionnelle autre que la poudre présentant une conductivité thermique inférieure ou égale à $2 \text{ W.m}^{-1}.\text{K}^{-1}$.

13. Utilisation selon l'une quelconque des revendications précédentes, **caractérisée en ce que** la composition cosmétique comprend au moins une matière colorante choisie parmi les pigments, les nacres et les colorants hydro ou liposolubles.

**Patentansprüche**

1. Verwendung von mindestens einem Pulver, das eine Wärmeleitfähigkeit von weniger als oder gleich $2 \text{ W.m}^{-1}.\text{K}^{-1}$, vorzugsweise zwischen 0 und $2 \text{ W.m}^{-1}.\text{K}^{-1}$, bevorzugter zwischen 0,1 und $1,5 \text{ W.m}^{-1}.\text{K}^{-1}$ aufweist, zur Verbesserung des thermischen Komforts, der von einer kosmetischen Zusammensetzung zum Schminken oder Pflegen der Haut verliehen wird.

2. Verwendung nach Anspruch 1, **dadurch gekennzeichnet, dass** die Verbesserung des thermischen Komforts durch Verringern der Sichtbarkeit der Poren unter Bedingungen, die für eine feuchte Wärme repräsentativ sind (zum Beispiel 30 °C und 75% relative Feuchtigkeit), oder durch Verringern der Rötungen unter Bedingungen einer trockenen Kälte (zum Beispiel 7 °C, 40% relative Feuchtigkeit) erreicht wird.

3. Verwendung nach einem der vorhergehenden Ansprüche, **dadurch gekennzeichnet, dass** das Pulver ein Partikel von sphärischer oder lamellarer Form ist, mineralisch oder organisch, unlöslich in der kosmetischen Zusammensetzung.

4. Verwendung nach einem der vorhergehenden Ansprüche, **dadurch gekennzeichnet, dass** das Pulver ausgewählt ist aus Talk, Siliziumdioxid, Aluminiumoxid, den Aluminiumsilikaten, insbesondere den Aluminiumborosilikaten wie den Glaskügelchen, Silicat von Magnesium und Aluminium wie Fluorphlogopit, Bentonit, mit $TiO_2$ und $SiO_2$ beschichteten Keramikmikrokugeln, Calciumphosphat wie Hydroxylapatit, Magnesiumsulfat, Magnesiumcarbonat, Kaolin, Bornitrid, Cellulose, Glimmer, PMMA-Pulver, den Polyamiden, den Silikonharze wie den Polymethylsilsesquioxanen, den Silikonelastomerpulvern, gegebenenfalls beschichtet mit einem Silikonharz, und ihren Mischungen.

5. Verwendung gemäß einem der vorhergehenden Ansprüche, **dadurch gekennzeichnet, dass** das Pulver ausgewählt ist aus Bornitrid, Talk, Glimmer, mit $TiO_2$ und $SiO_2$ beschichteten Keramikmikrokugeln, Glaskügelchen, Porzellan und ihren Mischungen.

6. Verwendung gemäß einem der vorhergehenden Ansprüche, **dadurch gekennzeichnet, dass** das Pulver ausgewählt ist aus Bornitrid, mit $TiO_2$ und $SiO_2$ beschichteten Keramikmikrokugeln und ihren Mischungen.

7. Verwendung gemäß einem der vorhergehenden Ansprüche, **dadurch gekennzeichnet, dass** die kosmetische Zusammensetzung, die das Pulver mit einer Wärmeleitfähigkeit von weniger als oder gleich 2 $W.m^{-1}.K^{-1}$ umfasst, in Form eines Pulvers, einer Emulsion, einer Mikroemulsion, einer Nanoemulsion, einer Suspension, einer Lösung, einer Lotion, einer Creme, eines wässrigen oder hydroalkoholischen Gels, eines Schaums, eines Serums, einer wasserfreien Zusammensetzung oder einer Dispersion für ein Aerosol vorliegt.

8. Verwendung nach einem der vorhergehenden Ansprüche, **dadurch gekennzeichnet, dass** die kosmetische Zusammensetzung, die das Pulver mit einer Wärmeleitfähigkeit von weniger als oder gleich 2 $W.m^{-1}.K^{-1}$ umfasst, in Form einer Fluidzusammensetzung vorliegt.

9. Verwendung nach einem der vorhergehenden Ansprüche, **dadurch gekennzeichnet, dass** die kosmetische Zusammensetzung, die das Pulver mit einer Wärmeleitfähigkeit von weniger als oder gleich 2 $W.m^{-1}.K^{-1}$ umfasst, in Form eines losen, kompakten oder gegossenen Pulvers, vorzugsweise eines kompakten Pulvers vorliegt.

10. Verwendung nach Anspruch 8, **dadurch gekennzeichnet, dass** das Pulver mit einer Wärmeleitfähigkeit von weniger als oder gleich 2 $W.m^{-1}.K^{-1}$ 0,1 bis 50 Gewichts-%, vorzugsweise 1 bis 20 Gewichts-% und bevorzugter 2 bis 15 Gewichts-%, bezogen auf das Gesamtgewicht der kosmetischen Zusammensetzung, die es umfasst, darstellt.

11. Verwendung nach Anspruch 9, **dadurch gekennzeichnet, dass** das Pulver mit einer Wärmeleitfähigkeit von weniger als oder gleich 2 $W.m^{-1}.K^{-1}$ 10 bis 90 Gewichts-%, vorzugsweise 20 bis 80 Gewichts-% und bevorzugter 35 bis 70 Gewichts-%, bezogen auf das Gesamtgewicht der kosmetischen Zusammensetzung, die es umfasst, darstellt.

12. Verwendung nach einem der vorhergehenden Ansprüche, **dadurch gekennzeichnet, dass** die kosmetische Zusammensetzung mindestens einen zusätzlichen Füllstoff umfasst, der ein anderer ist als das Pulver, aufweisend eine Wärmeleitfähigkeit von weniger als oder gleich 2 $W.m^{-1}.K^{-1}$.

13. Verwendung nach einem der vorhergehenden Ansprüche, **dadurch gekennzeichnet, dass** die kosmetische Zusammensetzung mindestens einen Farbstoff umfasst, ausgewählt aus den Pigmenten, den Perlmutten und den wasser- oder fettlöslichen Farbstoffen.

## Claims

1. The use of at least one powder having a thermal conductivity less than or equal to 2 $W.m^{-1}.K^{-1}$, preferably between 0 and 2 $W.m^{-1}.K^{-1}$, more preferably between 0.1 and 1.5 $W.m^{-1}.K^{-1}$ in order to improve the thermal comfort conferred by a cosmetic make-up or skincare composition.

2. The use according to Claim 1, **characterised in that** the improvement in thermal comfort is obtained by reducing the visibility of pores in conditions representative of a humid heat (for example, 30°C and 75% relative humidity), or by reducing redness under conditions of dry cold (for example, 7°C, 40% relative humidity).

3. The use according to any one of the preceding claims, **characterised in that** the powder is a mineral or organic particle having spherical or laminar shape, that is insoluble in the cosmetic composition.

4. The use according to any one of the preceding claims, **characterised in that** the powder is chosen from among talc, silica, alumina, aluminium silicates, in particular aluminium borosilicates such as glass balls, magnesium and aluminium silicate, such as fluorphlogopite, bentonite, ceramic microspheres coated with $TiO_2$ and $SiO_2$, calcium phosphate, such as hydroxyapatite, magnesium sulfate, magnesium carbonate, kaolin, boron nitride, cellulose, mica, PMMA powders, polyamides, silicone resins, such as polymethylsilsesquioxanes, silicone elastomer powders possibly coated by a silicone resin, and the mixtures thereof.

5. The use according to any one of the preceding claims, **characterised in that** the powder is chosen from among boron nitride, talc, mica, ceramic microspheres coated with $TiO_2$ and $SiO_2$, glass balls, porcelain, and the mixtures thereof.

6. The use according to any one of the preceding claims, **characterised in that** the powder is chosen from among boron nitride, ceramic microspheres coated with $TiO_2$ and $SiO_2$, and the mixtures thereof.

7. The use according to any one of the preceding claims, **characterised in that** the cosmetic composition comprising the powder with thermal conductivity less than or equal to 2 $W.m^{-1}.K^{-1}$ is in the form of a powder, an emulsion, a microemulsion, a nanoemulsion, a suspension, a solution, a lotion, a cream, an aqueous or hydroalcoholic gel, a foam, a serum, an anhydrous composition or an aerosol dispersion.

8. The use according to any one of the preceding claims, **characterised in that** the cosmetic composition comprising the powder with thermal conductivity less than or equal to 2 $W.m^{-1}.K^{-1}$ is in the form of a fluid composition.

9. The use according to any one of the preceding claims, **characterised in that** the cosmetic composition comprising the powder with thermal conductivity less than or equal to 2 $W.m^{-1}.K^{-1}$ is in the form of a free, compact or cast powder, preferably a compact powder.

10. The use according to Claim 8, **characterised in that** the powder with thermal conductivity less than or equal to 2 $W.m^{-1}.K^{-1}$ represents 0.1 to 50% by weight, preferably 1 to 20% by weight, and more preferably 2 to 15% by weight with respect to the total weight of the cosmetic composition comprising it.

11. The use according to Claim 9, **characterised in that** the powder with thermal conductivity less than or equal to 2 $W.m^{-1}.K^{-1}$ represents 10 to 90% by weight, preferably 20 to 80% by weight, and more preferably 35 to 70% by weight with respect to the total weight of the cosmetic composition comprising it.

12. The use according to any one of the preceding claims **characterised in that** the cosmetic composition comprises at least one additional filler other than the powder having a thermal conductivity less than or equal to 2 $W.m^{-1}.K^{-1}$.

13. The use according to any one of the preceding claims, **characterised in that** the cosmetic composition comprises at least one colouring material chosen from pigments, nacres and water-soluble or liposoluble colouring agents.

**Figure 1**

**Figure 2**

**Figure 3**

**Figure 4**

**EP 3 380 198 B1**

## RÉFÉRENCES CITÉES DANS LA DESCRIPTION

**Documents brevets cités dans la description**

- WO 2014133957 A
- WO 9838981 A
- US 6309629 B
- EP 542669 A
- EP 787730 A
- EP 787731 A
- WO 9608537 A
- EP 0518773 A